# EUROPEAN PATENT SPECIFICATION

(11) **EP 1 765 223 B1**
(45) Date of publication and mention of the grant of the patent: **24.07.2013**
(21) Application number: 05764479.1
(22) Date of filing: 30.06.2005
(51) Int. Cl.: A61F 2/958

(54) **BALLOON CATHETER SYSTEM FOR SECURING A STENT**
BALLONKATHETER UND SYSTEM ZUR FIXIERUNG EINES STENTS
CATHETER A BALLONNET ET SYSTEME PERMETTANT DE FIXER UNE ENDOPROTHESE

(30) Priority: 09.07.2004 US 586510 P
(43) Date of publication of application: 28.03.2007
(73) Proprietor: Conor Medsystems, Inc., Menlo Park, CA 94025 (US)
(72) Inventor: FISHER, Beau, M., Danville, CA 94526 (US)
(74) Representative: Schorr, Frank Jürgen
(86) International application number: PCT/US2005/023693
(87) International publication number: WO 2006/017057

(56) References cited:
- EP-A1- 1 132 059
- US-A1- 2003 208 254
- US-B1- 6 309 402
- US-B1- 6 464 718
- US-B1- 6 561 788
- US-B2- 6 863 683

## Description

### Background

The present invention relates to tissue-supporting medical devices and delivery systems. More particularly the invention relates to a balloon catheter and to a method and system for securing a stent to a balloon catheter.

Stents are expandable cylindrical devices that are implanted within a bodily lumen of a living animal or human to support the organ and maintain patency. Stents are typically introduced percutaneously, and transported transluminally until positioned at a desired location. The stent is then expanded either mechanically, generally by the expansion of a balloon positioned inside the stent to support the lumen. Once expanded within the lumen the stents become encapsulated within the body tissue. Stents can be biodegradable or remain a permanent implant.

Known stent designs include monofilament wire coil stents (U.S. Pat. No. 4,969,458); welded metal cages (U.S. Pat. Nos. 4,733,665 and 4,776,337); and, most prominently, thin-walled metal cylinders with axial slots formed around the circumference (U.S. Pat. Nos. 4,733,665, 4,739,762, and 4,776,337). Known construction materials for use in stents include polymers, organic fabrics, biocompatible metals, such as, stainless steel, gold, silver, tantalum, cobalt alloys, titanium, and shape memory alloys such as Nitinol, and biodegradable polymers and metal alloys.

U.S. Pat. Nos. 4,733,665; 4,739,762; 4,776,337; 6,241,762; and 6,562,065 disclose expandable and deformable stents in the form of thin-walled tubular members with axial slots allowing the members to be expanded radially outwardly by a balloon into contact with a body passageway.

Generally stents are delivered after dilation of a lumen by percutaneous transluminal angioplasty (PTA) or atherectomy. When stents are delivered without a predilation process, the process is called direct stenting. With either type of procedure it is important to retain the stent on the balloon catheter while the catheter is advanced through the body lumen to the location where the stent will be implanted. If the stent moves on the balloon or is dislodged from the balloon it may not be accurately delivered to the lumen. It is also important that, after expansion, the stent is no longer adhered to the balloon.

The systems for securing stents onto balloons for delivery include systems for crimping or compressing the stent onto the balloon to achieve adherence. Crimping is often used in combination with another technique to increase adherence. For example, stent securing systems including adhesives are described in U.S. Pat. Nos. 6,682,553 and 6,635,078. Other systems for improving adherence involve deformation of the balloon beneath the stent to extend portions of the balloon between cells in the stent creating improved retention. Examples of such balloon deformation systems are described in U.S. Pat. Nos. 6,309,402 and 6,666,880.

However, there is a need to retain stents more securely on a balloon catheter without using adhesives that may create difficulties in releasing the stent from the balloon and without adversely affecting any drug on the stent.

Document US 2003/0208254 A1 discloses a method and apparatus, wherein a stent/balloon assembly is positioned between two mould sections, pressure is applied, and the mould is heated to secure the stent to the balloon.

### Summary of the Invention

The present invention relates to a balloon catheter with a pillow and a method and system for securing a stent to a balloon catheter.

In accordance with one aspect of the invention, a balloon catheter/stent assembly according to claim 15 is provided.

In accordance with another aspect of the invention, a system according to claim 1 is provided.

In accordance with an additional aspect of the invention, a method according to claim 7 is provided.

### Brief Description of the Drawings

The invention will now be described in greater detail with reference to the preferred embodiments illustrated in the accompanying drawings, in which like elements bear like reference numerals, and wherein:
FIG. 1 is a side cross sectional view of a distal end of a stent delivery system including a balloon catheter and a stent, with the balloon catheter in a deflated condition and the stent in an unexpanded condition.
FIG. 2 is a side cross sectional view of the stent delivery system of FIG. 1 with the balloon catheter in an inflated condition and the stent in an expanded condition.
FIG. 3 is a side cross sectional view of a restrainer configured with the stent delivery system received in the lumen of the restrainer for formation of stent retaining pillows.
FIG. 4 is a schematic perspective view of a system for securing a stent to a balloon catheter.

### Detailed Description

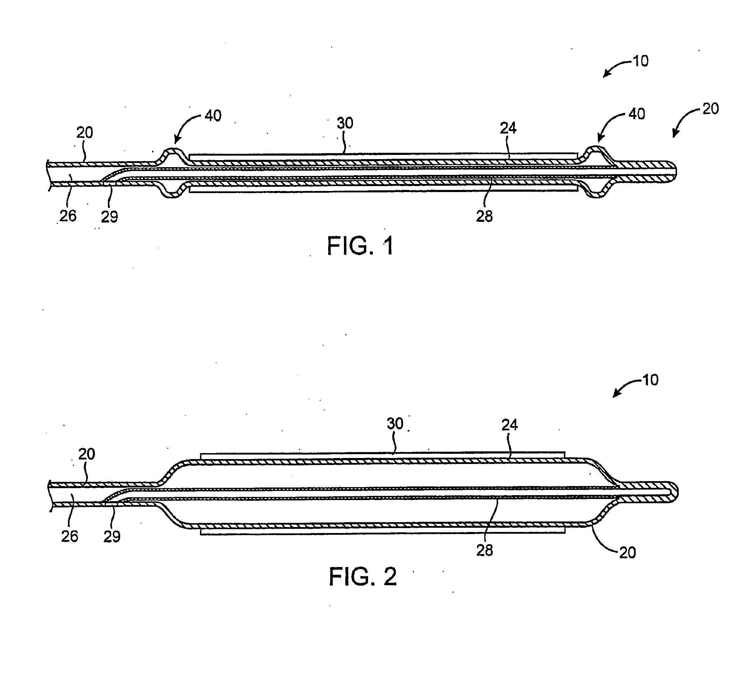

FIG. 1 illustrates a catheter/stent apparatus 10 including a balloon catheter 20 with a stent 30 mounted thereon. The balloon catheter 20 has an elongated catheter shaft 22, only a portion of which is shown. A balloon 24 is positioned at a distal end of the elongated catheter shaft 22 and an inflation lumen 26 extends within the elongated catheter shaft for inflation and deflation of the balloon.

The stent 30 which is mounted and crimped onto the balloon 24 may be any known stent, for example, the stent can be a drug delivery stent such as those illustrated in U.S. Patent Publication No. 2003/0199970, published on October 23, 2003 The stent 30 is positioned on the balloon 24 with a small portion of the balloon extending beyond each end of the stent. This extension of the balloon 24 beyond the end of the stent 30 helps to ensure complete expansion of the stent.

The catheter can be any of the known angioplasty or stent delivery catheters. As shown in FIGS. 1 and 2, the catheter 20 is preferably a rapid exchange catheter having a short guidewire lumen 28 beneath the balloon 24 and a proximal guidewire opening 29 which is closer to the balloon than to the proximal end of the catheter.

As shown in FIG. 1, at least one and preferably two pillows 40 are provided on the catheter balloon 24 between the ends of the stent 30 and the ends of the balloon. The pillows 40 are formed from the material of the expandable balloon 24 and are formed by expansion and heat setting the balloon after mounting and crimping the stent on the balloon. The pillows 40 extend radially outward from the outer surface of the uninflated balloon 24 to help to retain the stent 30 onto the balloon during stent insertion. The pillows 40 have an outer diameter which is larger than the outer diameter of the mounted and crimped stent 30. When the pillows 40 are formed adjacent to the ends of the stent 30 they improve stent retention over crimping alone.

As shown in FIG. 2, when the balloon 24 is expanded, the pillows 40 expand to essentially the same diameter as the remainder of the balloon 24 and essentially disappear.

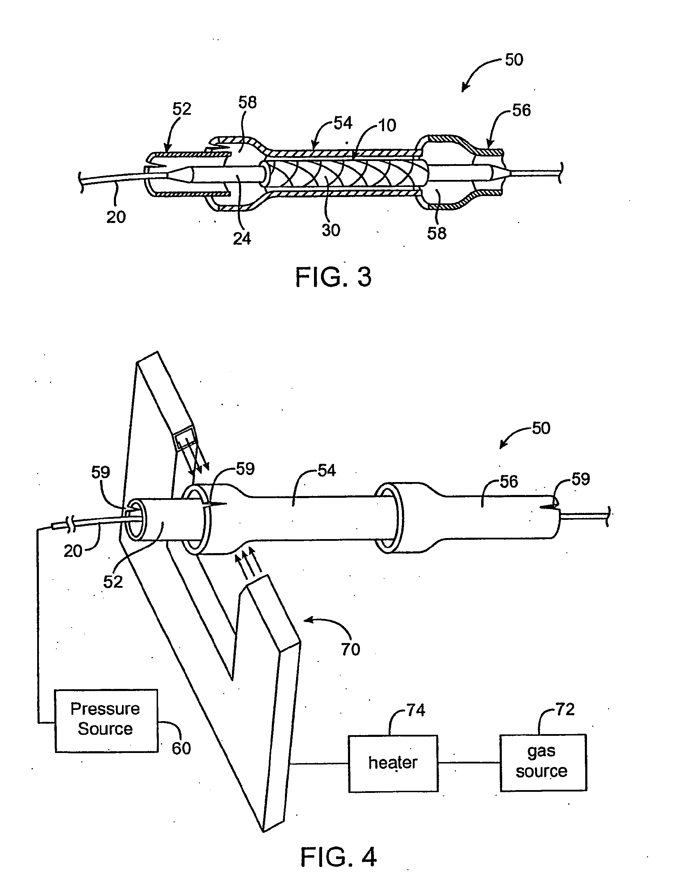

FIG. 3 shows a cut-away view of a system of sheaths which form a restrainer 50 over the catheter/stent assembly 10 for the pillowing process. The sheaths include a proximal sheath 52, a center sheath 54, and a distal sheath 56. The sheaths are assembled over the catheter/stent assembly 10 to form gaps 58 at the ends of the stent 30. Heat and pressure of the pillowing process will form the balloon 24 into these gaps to create the pillows 40 or small lumps that prevent the stent from sliding on the balloon 24 during use.

The proximal sheath 52 is a simple substantially cylindrical sheath having an inner diameter which is sufficiently large to fit over the catheter shaft and a portion of the end of the balloon 24 without the stent 30. The center sheath 54 is stepped with a first inner diameter which is approximately equal to an outer diameter of the crimped stent 30 and a second larger inner diameter which is greater than an outer diameter of the stent providing a step within the lumen of the sheath. The distal sheath 56 is also stepped with a first inner diameter which is the same as that of the proximal sheath 52 and a second larger diameter which is the same as the larger diameter of the center sheath 54. The sheaths are each slid over the catheter 20 and stent 30 and assembled in a manner such that the gaps 58 are formed precisely adjacent the ends of the stent for formation of the pillows 40.

The gaps 58 and the pillows which are formed in these gaps can have a length of about 0.5 to about 5.0 mm, preferably about 1 to about 2.5 mm. The gaps 58 are preferably formed to create a pillow having a height above the maximum crimped outer diameter of the stent which is approximately the same as the thickness of the stent wall. More particularly, the height which the pillow extends beyond the outer diameter of the crimped stent is about ½ to about 3 times the thickness of the stent wall. A difference between an outer diameter of the crimped stent and an outer diameter of the pillow can be about 0.01 to about 1.0 mm, preferably about 0.05 to about 0.3 mm, and more preferably about 0.1 to about 0.2 mm.

Each of the sheaths 52, 54, 56 can be provided with one or more slits 59 or perforations, i.e. two slits on one end of each sheath. The slits 59 allow the sheaths to be peeled off of the catheter/stent assembly 10 after formation of the pillows 40.

The multiple sheath system is used to allow the sheaths to be easily positioned over the catheter. Alternative sheath systems (not part of the invention) can also be used to form a restrainer, such as a single sheath with two steps or multiple sheaths that have been positioned partially coaxially to form the steps. One example of a multiple sheath system includes three small diameter sheaths placed with two of the sheaths proximal and distal of the pillow locations and a central sheath extending the length of the stent so that gaps occur between the small diameter sheaths at the locations of the pillows. A large diameter sheath can then be placed over the three small diameter sheaths to form the gaps into which the balloon is expanded to form the pillows.

As shown in FIG. 4, once the sheaths have been positioned over the catheter/stent assembly 10 the pillows can be expanded and set. The catheter is connected to a pressure source 60 for pressurization of the balloon. A pressure source 60 or pressure manifold provides a source of pressurized fluid. The pressurization of the balloon depends on the balloon material, construction, and size. According to one embodiment, the catheter is pressurized to between about 6.9 and 138 bar (100 and about 200 psi) preferably about 7.6 and 11.0 bar (110 to about 160 psi). The pressure is selected to be a pressure at which the balloon is expanded into the gaps 58 and is pressed into contact with the inner diameter of the sheaths to form the pillows 40.

The catheter is maintained pressurized while the pillows are heat set. The heat set process applies heat to the pillow areas of the catheter, without causing significant heating of the stent. Particularly in the mounting of a drug delivery stent, heating of the stent could cause degradation of the drug or melting of any polymer drug carrier. The application of heat to the pillow is performed as follows: A hot gas jet 70 is used as the heating device. The hot gas jet 70 aimed at the area to be heated provides heating of the pillow 40 without overheating the stent. In one example of a heating device, a gas source 72, such as nitrogen, air, or inert gas, provides gas to a heater 74, which heats the gas to be delivered by the hot gas jet 70. The hot gas jet can be integrated into the heater as a hot box. The hot gas jet 70 shown in FIG. 4 directs the hot gas at the catheter pillow area from two opposite directions. Other configurations of hot gas jets may include a single gas outlet or more than two gas outlets. The hot gas may be applied through a porous holding device, such as a foam pad.

The sheath system for retaining the catheter/stent assembly 10 during the pillowing procedure and for providing the gaps 58 into which the balloon expands to form the pillow is only one example of the type of retainer which can be used. Another retainer (not part of the invention) can include a mold which can be closed around the catheter/stent assembly and provides the gaps 58 for formation of the pillows. An adjustable mold can be used to accommodate different size stents.

The heating device including the hot gas jet 70 is an example of the type of heating devices that are used. Other heating devices such as a heating block (not part of the invention) can also be used. As long as the heat is concentrated at the area in which the pillow is to be formed, cooling of the adjacent stent area can be avoided. Shorter heating times also minimize any overheating of the stent. For example, the pillow areas can each be heated for about 20 to about 100 seconds, preferably about 25 to about 45 seconds. The hot gas temperature will be selected depending on the balloon material used. For example, a hot gas temperature of at least 79 °C (175 degrees F), and preferably about 93 °C (200 degrees F) is selected for a pillowing temperature for commonly used balloon of nylon or nylon-like materials.

Following the heating step, the formed pillows can be cooled by a cold jet or other cooling system while the internal catheter pressure is retained. Upon cooling, the catheter is removed from the pressure source or manifold and the sheaths are removed.

The final pillow configuration having a pillow diameter greater than a stent crimped diameter significantly improves retention of the stent 30 on the catheter 20. This improved retention is due to the fact that the stent 30 would have to expand beyond its crimped diameter to pass over the pillows 40. It has been found that pillows 40 extending out from the outer diameter of the stent a distance which is approximately equal to the thickness of the stent walls do not have any noticeable affect on the ability of the balloon catheter to deliver the stent to a target site through narrowed vessels. In fact, the balloon pillows 40 are sufficiently pliable that they compress during stent delivery when the catheter passes through tight spaces.

The stent according to the present invention can be used for supporting a variety of ducts or lumens within the body, such as coronary or peripheral arteries, lumens of the tracheal or bronchial tree, billiary ducts, and the like.

## Claims

1. A system for securing a stent to a balloon catheter, the system comprising:
a restrainer (50) having a restrainer lumen configured to receive a balloon catheter (20) with a stent (30) crimped thereon, the restrainer having a first inner diameter which is approximately equal to an outer diameter of the crimped stent and a second inner diameter which is greater than an outer diameter of the crimped stent providing a step within the lumen of the restrainer;
a catheter pressuring device (60) for pressurizing the balloon within the restrainer to form at least one pillow (40) adjacent to an end of the stent, the at least one pillow having an outer diameter larger than the outer diameter of the crimped stent; and
**characterized by**
a heating device (70) for heating the at least one pillow to set the pillow shape, wherein the heating device (70) is a hot gas nozzle configured to direct hot gas at the at least one pillow without substantially heating the crimped stent,
wherein the restrainer (50) comprises a first sheath providing the step within a lumen of the sheath,
wherein the restrainer (50) further comprises a second sheath having a step within a lumen and a largest inner diameter substantially the same as the second inner diameter of the first sheath.

2. The system of Claim 1, wherein the heating device (70) is configured to heat the at least one pillow to a temperature of at least 79.4°C (175 degrees F).

3. The system of Claim 1, wherein the catheter pressurizing device (60) is configured to pressurize the catheter to 7.6 to 11.0 bar (about 110 to 160 psi).

4. The system of Claim 1, wherein the restrainer (50) is configured to create a pillow having an outer diameter which is at least 0.05 mm larger than the crimped stent.

5. The system of Claim 1, wherein the restrainer (50) is configured to create a pillow having a length of about 0.5 mm to about 5 mm.

6. The system of Claim 1, wherein the at least one pillow comprises two pillows (40), with one pillow adjacent each opposite end of the stent (30).

7. A method of forming a catheter/stent assembly (10), the method comprising:
crimping a stent onto a balloon of a balloon catheter;
positioning at least a portion of the crimped stent and balloon into a restrainer having a first inner diameter which is approximately equal to an outer diameter of the crimped stent and a second inner diameter which is greater than an outer diameter, wherein the portion of the restrainer with the first inner diameter is placed around the stent and the portion of the restrainer with the second inner diameter is positioned adjacent an end of the stent;
pressurizing the balloon within the restrainer to form at least one pillow adjacent to an end of the stent, the at least one pillow having an outer diameter larger than the outer diameter of the crimped stent; and **characterized by**
heating the at least one pillow to set the pillow shape without substantially heating the crimped stent, wherein the heating is performed by a hot gas nozzle directing hot gas at the at least one pillow, wherein the restrainer comprises a first sheath providing a step within a lumen of the sheath,
wherein the restrainer further comprises a second sheath having a step within a lumen and inner diameters substantially the same as the first sheath.

8. The method of Claim 7, wherein the heating step comprises heating the at least one pillow to a temperature of at least 79.4°C (175 degrees F).

9. The method of Claim 7, wherein the pressurizing step comprises pressurizing the catheter to 7.6 to 11.0 bar (about 110 to 160 psi).

10. The method of Claim 7, wherein the pillow has an outer diameter which is at least 0.05 mm larger than the crimped stent.

11. The method of Claim 7, wherein the restrainer creates a pillow having a length of about 0.5 mm to about 5 mm.

12. The method of Claim 7, wherein the at least one pillow comprises two pillows, with one pillow formed adjacent each opposite end of the stent.

13. The method of Claim 7, further comprising removing the stent delivery system and stent from the restrainer.

14. The method of Claim 13, further comprising cooling the at least one pillow prior to removing the stent delivery system and stent from the restrainer.

15. A balloon catheter/stent assembly (10) obtained by the method of one of claims 7 to 14, the balloon catheter /stent assembly comprising:
a balloon catheter (20) having an elongated catheter shaft (22), a balloon (24) positioned at a distal end of the elongated catheter shaft, and an inflation lumen (26) extending within the elongated catheter shaft (22) for inflation and deflation of the balloon;
a stent (30) mounted and crimped onto the balloon, the mounted and crimped stent having an outer diameter;
and at least one pillow (40) on the catheter balloon, the at least one pillow formed from the balloon and having an outer diameter larger than the outer diameter of the mounted and crimped stent,
**characterized in that**
the at least one pillow is heat-set adjacent to an end of the stent, wherein the stent (30) comprises a heat degradable drug or polymer drug carrier.

16. The assembly of Claim 15, wherein a difference between the outer diameter of the mounted and crimped stent (30) and the outer diameter of the at least one pillow (40) is at least 0.05 mm.

17. The assembly of Claim 15, wherein a difference between the outer diameter of the mounted and crimped stent (30) and the outer diameter of the at least one pillow (40) is about a thickness of the stent or greater.

18. The assembly of Claim 15, wherein the at least one pillow (40) has a length of 0.5 mm to 5 mm.

19. The assembly of Claim 15, wherein the at least one pillow comprises two pillows (40), with one pillow adjacent each opposite end of the stent (30).

20. The assembly of Claim 15, wherein the at least one pillow comprises a continuous portion of the balloon which has a larger diameter than a remainder of the balloon created by pressurization and heat setting.

21. The assembly of Claim 15, wherein the at least one pillow (40) is compliant and compresses to pass through tight lesions.

22. The assembly of Claim 15, wherein the at least one pillow (40) prevents the stent (30) from moving longitudinally on the balloon prior to expansion of the stent and allows the stent to move longitudinally on the balloon upon expansion of the stent.

23. The assembly of Claim 15, wherein the balloon catheter (20) is a rapid exchange catheter.

## Patentansprüche

1. System zum Befestigen eines Stents an einem Ballonkatheter, wobei das System Folgendes umfasst:
ein Rückhalteelement (50), das ein Rückhalteelementlumen aufweist, welches konfiguriert ist, um einen Ballonkatheter (20) mit einem darauf geklemmten Stent (30) aufzunehmen, wobei das Rückhalteelement einen ersten Innendurchmesser, der etwa gleich wie ein Außendurchmesser des geklemmten Stents ist, und einen zweiten Innendurchmesser, der größer als ein Außendurchmesser des geklemmten Stents ist, aufweist, wodurch in dem Lumen des Rückhalteelements eine Stufe bereitgestellt wird;
eine Katheter-Unterdrucksetzungsvorrichtung (60) zum Unterdrucksetzen des Ballons innerhalb des Rückhalteelements, um neben einem Ende des Stents mindestens ein Kissen (40) zu bilden, wobei das mindestens eine Kissen einen Außendurchmesser aufweist, der größer ist als der Außendurchmesser des geklemmten Stents; und
**gekennzeichnet ist durch**
eine Heizvorrichtung (70) zum Erwärmen des mindestens einen Kissens zum Fixieren der Kissenform, wobei es sich bei der Heizvorrichtung (70) um eine Heißgasdüse handelt, die konfiguriert ist, um heißes Gas auf das mindestens eine Kissen zu richten, ohne den geklemmten Stent wesentlich zu erwärmen,
wobei das Rückhalteelement (50) eine erste Ummantelung aufweist, welche die Stufe in einem Lumen der Ummantelung bereitstellt,
wobei das Rückhalteelement (50) ferner eine zweite Ummantelung aufweist, die in einem Lumen eine Stufe und einen größten Innendurchmesser aufweist, der im Wesentlichen gleich wie der zweite Innendurchmesser der ersten Ummantelung ist.

2. System gemäß Anspruch 1, wobei die Heizvorrichtung (70) konfiguriert ist, um das mindestens eine Kissen auf eine Temperatur von mindestens 79,4 °C (175 Grad Fahrenheit) zu erwärmen.

3. System gemäß Anspruch 1, wobei die Katheter-Unterdrucksetzungsvorrichtung (60) konfiguriert ist, um den Katheter unter einen Druck von 7,6 bis 11,0 bar (etwa 110 bis 160 psi) zu setzen.

4. System gemäß Anspruch 1, wobei das Rückhalteelement (50) konfiguriert ist, um ein Kissen zu erzeugen, das einen Außendurchmesser aufweist, der mindestens 0,05 mm größer ist als der geklemmte Stent.

5. System gemäß Anspruch 1, wobei das Rückhalteelement (50) konfiguriert ist, um ein Kissen zu erzeugen, das eine Länge von etwa 0,5 mm bis etwa 5 mm aufweist.

6. System gemäß Anspruch 1, wobei das mindestens eine Kissen zwei Kissen (40) umfasst, wobei sich neben jedem gegenüber liegenden Ende des Stents (30) ein Kissen befindet.

7. Verfahren des Bildens einer Katheter/Stent-Anordnung (10), wobei das Verfahren Folgendes umfasst:
Bördeln eines Stents auf einen Ballon eines Ballonkatheters;
Positionieren mindestens eines Abschnitts des geklemmten Stents und des Ballons in ein Rückhalteelement, das einen ersten Innendurchmesser, der etwa gleich wie ein Außendurchmesser des geklemmten Stents ist, und einen zweiten Innendurchmesser, der größer als ein Außendurchmesser ist, aufweist, wobei der Abschnitt des Rückhalteelements mit dem ersten Innendurchmesser um den Stent angeordnet wird und der Abschnitt des Rückhalteelements mit dem zweiten Innendurchmesser neben einem Ende des Stents positioniert wird;
Unterdrucksetzen des Ballons in dem Rückhalteelement, um mindestens ein Kissen neben einem Ende des Stents zu bilden, wobei das mindestens eine Kissen einen Außendurchmesser aufweist, der größer als der Außendurchmesser des geklemmten Stents ist;
und **gekennzeichnet ist durch**
Erwärmen des mindestens einen Kissens, um die Kissenform zu fixieren, ohne den geklemmten Stent wesentlich zu erwärmen, wobei das Erwärmen von einer Heißgasdüse durchgeführt wird, die heißes Gas auf das mindestens eine Kissen richtet, wobei das Rückhalteelement eine erste Ummantelung umfasst, welche in einem Lumen der Ummantelung eine Stufe bereitstellt,
wobei das Rückhalteelement ferner eine zweite Ummantelung aufweist, die in einem Lumen eine Stufe und Innendurchmesser aufweist, die im Wesentlichen gleich sind wie bei der ersten Ummantelung.

8. Verfahren gemäß Anspruch 7, wobei der Erwärmungsschritt das Erwärmen des mindestens einen Kissens auf eine Temperatur von mindestens 79,4 °C (175 Grad Fahrenheit) umfasst.

9. Verfahren gemäß Anspruch 7, wobei der Unterdrucksetzungsschritt das Unterdrucksetzen des Katheters auf 7,6 bis 11,0 bar (etwa 110 bis 160 psi) umfasst.

10. Verfahren gemäß Anspruch 7, wobei das Kissen einen Außendurchmesser aufweist, der mindestens 0,05 mm größer ist als der geklemmte Stent.

11. Verfahren gemäß Anspruch 7, wobei das Rückhalteelement ein Kissen erzeugt, das eine Länge von etwa 0,5 mm bis etwa 5 mm aufweist.

12. Verfahren gemäß Anspruch 7, wobei das mindestens eine Kissen zwei Kissen umfasst, wobei neben jedem gegenüber liegenden Ende des Stents ein Kissen geformt ist.

13. Verfahren gemäß Anspruch 7, welches ferner das Entfernen des Stenteinführsystems und des Stents von dem Rückhalteelement umfasst.

14. Verfahren gemäß Anspruch 13, welches ferner das Kühlen des mindestens einen Kissens vor dem Entfernen des Stenteinführsystems und des Stents von dem Rückhalteelement umfasst.

15. Ballonkatheter/Stent-Anordnung (10), die mit dem Verfahren gemäß eines der Ansprüche 7 bis 14 erhalten wird, wobei die Ballonkatheter/Stent-Anordnung Folgendes umfasst:
einen Ballonkatheter (20), der einen länglichen Katheterschaft (22), einen an einem distalen Ende des länglichen Katheterschaftes positionierten Ballon (24) und ein Füllungslumen (26) zum Füllen und Entleeren des Ballons, das in dem länglichen Katheterschaft (22) verläuft, aufweist;
einen auf den Ballon montierten und geklemmten Stent (30), wobei der montierte und geklemmte Stent einen Außendurchmesser aufweist;
und mindestens ein Kissen (40) auf dem Katheterballon, das mindestens eine Kissen aus dem Ballon geformt ist und einen Außendurchmesser aufweist, der größer ist als der Außendurchmesser des montierten und geklemmten Stents;
**dadurch gekennzeichnet, dass**
das mindestens eine Kissen neben einem Ende des Stents thermofixiert wird, wobei der Stent (30) einen durch Wärme abbaubaren Wirkstoff- oder Polymerwirkstoffträger umfasst.

16. Anordnung gemäß Anspruch 15, wobei ein Unterschied zwischen dem Außendurchmesser des montierten und geklemmten Stents (30) und dem Außendurchmesser des mindestens einen Kissens (40) mindestens 0,05 mm beträgt.

17. Anordnung gemäß Anspruch 15, wobei ein Unterschied zwischen dem Außendurchmesser des montierten und geklemmten Stents (30) und dem Außendurchmesser des mindestens einen Kissens (40) etwa eine Dicke des Stents beträgt oder größer ist.

18. Anordnung gemäß Anspruch 15, wobei das mindestens eine Kissen (40) eine Länge von 0,5 mm bis 5 mm aufweist.

19. Anordnung gemäß Anspruch 15, wobei das mindestens eine Kissen zwei Kissen (40) umfasst, wobei sich neben jedem gegenüber liegenden Ende des Stents (30) ein Kissen befindet.

20. Anordnung gemäß Anspruch 15, wobei das mindestens eine Kissen einen fortlaufenden Abschnitt des Ballons aufweist, der einen größeren Durchmesser als ein Rest des Ballons aufweist, der durch Unterdrucksetzen und Thermofixierung erzeugt wird.

21. Anordnung gemäß Anspruch 15, wobei das mindestens eine Kissen (40) nachgiebig ist und sich komprimiert, um durch enge Läsionen zu passen.

22. Anordnung gemäß Anspruch 15, wobei das mindestens eine Kissen (40) verhindert, dass der Stent (30) sich vor der Expansion des Stents in Längsrichtung auf dem Ballon verschiebt, und es dem Stent ermöglicht, sich bei Expansion des Stents in Längsrichtung auf dem Ballon zu verschieben.

23. Anordnung gemäß Anspruch 15, wobei es sich bei dem Ballonkatheter (20) um einen Schnellwechselkatheter handelt.

## Revendications

1. Système pour fixer un stent sur un cathéter à ballon, le système comprenant:
un dispositif de retenue (50) ayant une lumière de retenue configurée pour recevoir un cathéter à ballon (20) avec un stent (30) serti sur ce dernier, le dispositif de retenue ayant un premier diamètre interne qui est approximativement égal à un diamètre externe du stent serti et un second diamètre interne qui est supérieur à un diamètre externe du stent serti fournissant un échelon à l'intérieur de la lumière du dispositif de retenue;
un dispositif de mise sous pression de cathéter (60) pour mettre sous pression le ballon à l'intérieur du dispositif de retenue afin de former au moins un coussin (40) adjacent à une extrémité du stent, l'au moins un coussin ayant un diamètre externe supérieur au diamètre externe du stent serti; et
**caractérisé par**:
un dispositif de chauffage (70) pour chauffer l'au moins un coussin afin de déterminer la forme du coussin, dans lequel le dispositif de chauffage (70) est une buse de gaz chaud configurée pour diriger du gaz chaud au niveau de l'au moins un coussin sans chauffer sensiblement le stent serti,
dans lequel le dispositif de retenue (50) comprend une première gaine fournissant l'échelon à l'intérieur d'une lumière de la gaine,
dans lequel le dispositif de retenue (50) comprend en outre une seconde gaine ayant un échelon à l'intérieur d'une lumière et un plus grand diamètre interne sensiblement identique au second diamètre interne de la première gaine.

2. Système selon la revendication 1, dans lequel le dispositif de chauffage (70) est configuré pour chauffer l'au moins un coussin à une température d'au moins 79,4°C (175 degrés F).

3. Système selon la revendication 1, dans lequel le dispositif de mise sous pression de cathéter (60) est configuré pour mettre sous pression le cathéter de 7,6 à 11,0 bar (environ 110 à 160 psi).

4. Système selon la revendication 1, dans lequel le dispositif de retenue (50) est configuré pour créer un coussin ayant un diamètre externe qui est au moins plus grand de 0,05 mm que le stent serti.

5. Système selon la revendication 1, dans lequel le dispositif de retenue (50) est configuré pour créer un coussin ayant une longueur d'environ 0,5 mm à environ 5 mm.

6. Système selon la revendication 1, dans lequel l'au moins un coussin comprend deux coussins (40), avec un coussin adjacent à chaque extrémité opposée du stent (30).

7. Procédé pour former un ensemble de cathéter / stent (10), le procédé comprenant les étapes consistant à:
sertir un stent sur un ballon d'un cathéter à ballon;
positionner au moins une partie du stent serti et du ballon dans un dispositif de retenue ayant un premier diamètre interne qui est approximativement égal à un diamètre externe du stent serti et un second diamètre interne qui est supérieur à un diamètre externe, dans lequel la partie du dispositif de retenue avec le premier diamètre interne est placée autour du stent et la partie du dispositif de retenue avec le second diamètre interne est positionnée de manière adjacente à une extrémité du stent;
mettre sous pression le ballon à l'intérieur du dispositif de retenue afin de former au moins un coussin adjacent à une extrémité du stent, l'au moins un coussin ayant un diamètre externe plus grand que le diamètre externe du stent serti; et **caractérisé par** l'étape consistant à:
chauffer l'au moins un coussin pour déterminer la forme du coussin sans chauffer sensiblement le stent serti, dans lequel l'étape de chauffage est réalisée par une buse de gaz chaud dirigeant le gaz chaud au niveau de l'au moins un coussin, dans lequel le dispositif de retenue comprend une première gaine fournissant un échelon à l'intérieur d'une lumière de la gaine,
dans lequel le dispositif de retenue comprend en outre une seconde gaine ayant un échelon à l'intérieur d'une lumière et des diamètres internes sensiblement identiques à la première gaine.

8. Procédé selon la revendication 7, dans lequel l'étape de chauffage comprend l'étape consistant à chauffer le au moins un coussin à une température d'au moins 79,4°C (175 degrés F).

9. Procédé selon la revendication 7, dans lequel l'étape de mise sous pression comprend l'étape consistant à mettre sous pression le cathéter, selon une pression de 7,6 à 11,0 bar (environ 110 à 160 psi).

10. Procédé selon la revendication 7, dans lequel le coussin a un diamètre externe qui est au moins 0,05 mm plus grand que le stent serti.

11. Procédé selon la revendication 7, dans lequel le dispositif de retenue crée un coussin ayant une longueur d'environ 0,5 mm à environ 5 mm.

12. Procédé selon la revendication 7, dans lequel l'au moins un coussin comprend deux coussins, avec un coussin formé de manière adjacente à chaque extrémité opposée du stent.

13. Procédé selon la revendication 7, comprenant e outre l'étape consistant à retirer le système de pose de stent et le stent du dispositif de retenue.

14. Procédé selon la revendication 13, comprenant en outre l'étape consistant à refroidir l'au moins un coussin avant de retirer le système de pose de stent et le stent du dispositif de retenue.

15. Ensemble de stent / cathéter à ballon (10) obtenu par le procédé selon l'une quelconque des revendications 7 à 14, l'ensemble de stent / cathéter à ballon comprenant:
un cathéter à ballon (20) ayant une tige de cathéter allongée (22), un ballon (24) positionné au niveau d'une extrémité distale de la tige de cathéter allongée, et une lumière de gonflage (26) s'étendant à l'intérieur de la tige de cathéter allongée (22) pour le gonflage et le dégonflage du ballon;
un stent (30) monté et serti sur le ballon, le stent monté et serti ayant un diamètre externe;
et au moins un coussin (40) sur le ballon du cathéter, l'au moins un coussin étant formé à partir du ballon et ayant un diamètre externe plus grand que le diamètre externe du stent monté et serti,
**caractérisé en ce que**:
l'au moins un coussin est thermofixé à côté d'une extrémité du stent, dans lequel le stent (30) comprend un médicament thermo-dégradable ou un support de médicament polymère.

16. Ensemble selon la revendication 15, dans lequel une différence entre le diamètre externe du stent monté et serti (30) et le diamètre externe de l'au moins un coussin (40) est au moins de 0,05 mm.

17. Ensemble selon la revendication 15, dans lequel une différence entre le diamètre externe du stent monté et serti (30) et le diamètre externe du au moins un coussin (40) représente environ une épaisseur du stent ou plus.

18. Ensemble selon la revendication 15, dans lequel l'au moins un coussin (40) a une longueur de 0, 5 mm à 5 mm.

19. Ensemble selon la revendication 15, dans lequel l'au moins un coussin comprend deux coussins (40), avec un coussin adjacent à chaque extrémité opposée du stent (30).

20. Ensemble selon la revendication 15, dans lequel l'au moins un coussin comprend une partie continue du ballon qui a un plus grand diamètre qu'un reste du ballonnet créé par pressurisation et thermofixation.

21. Ensemble selon la revendication 15, dans lequel l'au moins un coussin (40) est souple et se comprime pour passer à travers des lésions étroites.

22. Ensemble selon la revendication 15, dans lequel l'au moins un coussin (40) empêche le stent (30) de se déplacer longitudinalement sur le ballon avant expansion du stent et permet au stent de se déplacer longitudinalement sur le ballon suite à l'expansion du stent.

23. Ensemble selon la revendication 15, dans lequel le cathéter à ballon (20) est un cathéter à échange rapide.
